# EUROPEAN PATENT APPLICATION

(11) **EP 0 940 678 A1**
(43) Date of publication of application: **08.09.1999**
(21) Application number: 98200634.8
(22) Date of filing: 02.03.1998
(51) Int. Cl.: G01N 33/00, A61B 10/00

(54) **Analytical test device**

(71) Applicant: Akzo Nobel N.V., 6824 BM Arnhem (NL)
(72) Inventor: The designation of the inventor has not yet been filed
(74) Representative: Van Gent, Marieke

(57) **Abstract**

An analytical test device is described especially intended for home use. The device may be used to obtain samples from a body cavity, extract the obtained sample and analyse the sample for the presence of a certain analyte. Examples are provided for an analytical test device suited to determine the presence of the fungus Candida in vaginal samples as an aid in the diagnosis of Candidiosis.

## Description

The invention relates to an analytical test device specifically intended for home use, for determining the presence or amount of an analyte in a sample taken from a body cavity.

The need for a rapid and accurate diagnosis to ensure appropriate treatment by health care professionals is apparent. Diagnostic tests which determine the presence or amount of an analyte currently facilitate many diagnostic procedures. A rapid, sensitive, specific and simplistic assay is extremely useful for emergency situations, field testing, physicians offices and in home diagnostics, provided it can be performed easily, gives adequate results and is safe to handle by the user. Assays that require multiple incubation steps, handling of hazardous materials, lengthy procedures, special storage conditions or extraordinary skills are better performed by trained laboratory personnel.

Among those assays that made a successful entry in the home testing market are the home pregnancy and ovulation tests. These fulfil the above requirements in that they are safe to handle, easy to use and give adequate results. Most home pregnancy tests use a readily obtainable sample such as urine, which may contribute significantly to their success, when compared to devices that use other bodily fluids that are more difficult to obtain such as blood.

Devices for diagnostic assays that require samples that are even more difficult to obtain, such as samples to be taken from body cavities have also been described. In general, these assays consist of two parts, a sample taker part and a diagnostic assay. Many of the devices intended for home use provide the user with a sample taker and means for the transport of the sample to a laboratory, where the diagnostic assay is then to be performed

Such devices are typically provided with a swab, suited to take a sample from a particular body cavity. If the analyte to be detected is a micro-organism, the sample is then transferred to a suitable medium to support growth of that micro-organism. In case the micro-organism is a bacterium, growth might take several days, whereas culturing a virus may take two weeks. After that, the micro-organism has to be identified by biochemical, chromogenic or microscopic methods. Examples of such devices are described in US patents US 4,778,758, US 3,966,558, US 3,883,396, US 4,465,078 and EP 095,630.

Some analytes are abundantly present in samples taken from body cavities, in many cases even in sufficient quantities to allow simple and rapid detection without prior culturing or other amplification steps. US 4,562,043 describes a swab device for detecting occult blood in a faecal sample by a chromogenic method. US 4,355,113 and US 3,954,563 describe a swab device for self-detection of Gonorrhoea in males and females respectively with a colour indicator next to the swab and a saline ampoule for release after the sample has been taken. Such simple staining techniques however do not provide sufficient specificity for the majority of micro-organisms.

It has been appreciated in the art that immunological methods provide a more rapid and convenient method for the detection of micro-organisms when compared with chromogenic methods. Moreover, immunological methods usually provide more specific information about the analyte than chromogenic methods. The high sensitivity of immunological methods often allows the direct detection of micro-organisms or other analytes in cases where chromogenic methods would require prior culturing or other amplification steps.

Immunological methods may conveniently be performed in solution or suspension. Samples taken from body cavities rarely contain sufficient fluid for direct use in an immunoassay and therefore analytes are usually released from the sample taker by placing the sample taker in a solution. It may be necessary to use detergents, mechanical forces, enzymes or acids to solubilize the antigen. The extracted material may then be used in an immuno assay by mixing it with other reagents.

A device where the process of taking a sample from a body cavity as well as the diagnostic assay may be performed at home; is described in US patent 5,250,412 wherein an immunoassay is described for an analyte obtained with a swab, in which the analyte is then absorbed. Detection of specific analyte is performed by first immersing the swab in a solution containing a fluorescent antibody and subsequently by washing the swab in an aqueous wash fluid. Reading the result requires the swab being illuminated by UV light. This method requires the user to handle different potentially harmful reagents and also requires the availability of a UV light source.

PCT patent application WO 88/04431 also describes a swab device where the sample collector is pushed through different chambers containing reagents for detection of the analyte while on the swab. This method requires the user to perform a manifold of complex manipulation steps and is therefore error-prone.

It is the object of the present invention to provide an analytical test device for determining the presence or amount of an analyte in a sample taken from a body cavity that is easier to operate than those mentioned above requiring less handling steps, no further equipment and no specific skills beyond the ordinary, making it especially suited for home use.

This is achieved with a device, comprising:
a) a sample taker (10) with dimensions suited to fit into the body cavity and provided with means (11) to collect and withdraw the sample from the interior of the body cavity, said sample taker being attached to
b) a housing (20) for a test strip (30), said test strip comprising immunological means for detecting the analyte and
c) a separate container (40) in which the sample taker fits closely, suited to hold an extraction fluid (50) in such a way that the extraction fluid contacts the sample when the sample taker is fitted into the container and
d) means (60, 61, 62) to allow for the autonomous transport and application of material from the sample suspended in the extraction fluid onto the test strip.

Analytes may be bacteria, viruses, fungi or molecules and structures that constitute analysable parts thereof, but analytes may also be molecules not foreign to the human or animal body such as for instance secreted proteins, immunoglobulines, hormones and the like.

The device according to the present invention is especially useful for the detection of a fungus, for instance of the genus Candida, such as the species C. albicans, C. parapsilosis, C. tropicalis and/or C. glabrata.

With the word sample we refer to any material that can be obtained from body cavities, specifically bodily excretion products like saliva, urine or stool, or samples from oral, vaginal or intestinal mucous membranes, undefined compositions like vaginal smears, excretions of the outer ear, the nose or fluids in wounds. The device according to the invention is especially suited for the detection of an analyte in a sample such as a vaginal smear taken from a human female.

A body cavity may be any cavity of the human or animal body, especially the natural cavities such as mouth, throat, ear, vagina, cervix, rectum, urethra or cloaca, but cavity may also refer to non-naturally occurring openings such as wounds.

With the sample taker (10) a sample can be taken from the body cavity of interest. The sample taker may comprise a longitudinal body (17) having an anterior and a distal end with a housing attached to the distal end and also comprises means to collect and withdraw the sample (11) attached to the anterior end. The longitudinal body may be dimensioned to be received in the vagina of a human female positioning the means to collect and withdraw the sample at the appropriate location of the vagina where the sample is to be taken.

Means to collect and withdraw the sample from the interior of the body cavity may comprise absorbing material with for instance bibulous, sponge-like or fibrous characteristics. Alternatively, the means to collect and withdraw the sample may essentially consist of non-absorbing material. In this latter case, said means to collect and withdraw the sample may be provided with mechanical means like openings, notches, excavations, intrusions or protrusions, all designed to retain sufficient material when inserted into the body cavity.

Preferably, the sample taker and means to collect and withdraw the sample essentially consist of a non-absorbing, non-degradable, inert, synthetic material such as a synthetic polymer, more preferably polystyrene, polypropylene, polyethylene or ethylene vinylacetate.

In a most preferred embodiment, the sample taker comprises means to collect and withdraw the sample (11) which comprise a rounded head, thicker than the longitudinal body (17) of the sample taker, said head having a front end (13) and a distal end (14), said distal end being attached to the sample taker, the border between said ends being formed by the largest diameter of the head (15), wherein the distal end comprises notches (12) dimensioned to receive sufficient sample volume, said head also containing fluid channels (16) with openings at said front end of the head and at said notches, said openings at said notches being dimensioned such that the sample is released from the notches when fluid is applied under slight pressure at the opening of said channels at the front end of the head.

Housing refers to a hollow casing suited to hold a test strip (30) and protect said strip from being mechanically or chemically damaged or contaminated. The housing preferably comprises openings through which the results of an immunological reaction on the test strip can be read. The housing may also comprise an opening (21) for reading the result of the immunological reaction with the analyte and another opening for reading the result of an immunological integrity control reaction. Also, the housing may advantageously provide a handle with which the sample taker is to be manipulated when inserted into the body cavity.

Preferably the holding device comprises also an absorber (34), which is located downstream adjacent to the test strip, to remove excess test liquid. The material of the absorber is not critical and can be any material that is capable of absorbing liquids, for example a paper-like material or sponge-like materials. Advantageously the test strip can be extended so that the strip itself can function as an absorber. In the latter case no additional absorber is needed. In order to gain space this extended test strip can be rolled or zigzag-folded.

Besides the absorber the holding device may contain a moisture-absorbing agent (35), such as silica, to guarantee a better stability of the analytical system on the test strip. This moisture-absorbing agent should not be in contact with the test strip.

A test strip is a porous carrier through which a liquid is to be transported, said liquid comprising the analyte dissolved or suspended in extraction fluid, said carrier having an antibody specific for the analyte attached thereto, whereby part of said antibody is provided with a label and freely mobile within the porous carrier when in the moist state, and the other part of said antibody is immobilised in a detection zone on said porous carrier, the positioning of the labelled and immobilised antibody being such that test sample, when applied to the porous carrier, will first pick up the labelled antibody and thereafter will migrate to the detection zone. The labelled antibody and the freely mobile antibody may be of identical or of different specificity.

The test strip advantageously consists of a material which transports the test liquid essentially by capillary forces. Advantageously absorbent, porous or fibrous material is used, which is suitable for rapid uptake of liquid. Suitable materials involve amorphous sponge-like structures or various porous synthetic materials such as polypropylene, polyethylene, polyvinylidene fluoride, ethylene vinylacetate copolymer, polyacrylonitrile and polytetrafluoroethylene. In addition certain materials with an inherent hydrophobicity can be pre-treated with surface-active agents to such an extent that they are able to take up a test liquid and transport it by capillary forces. Other suitable materials include multilayer materials and materials which are already generally used in analytical test strips such as paper or paper-like materials, for example nitro-cellulose.

There are no special restrictions with respect to the analytical test system on the test strip other than that it is based on an immunological reaction. An example of an analytical system is described in German Utility Model DE-U-88 05 565.5. Other variants are to be found in EP-A-0 183 442, WO-A-91/12528, EP-A-0 349 215 and EP-A-0 186 799. This immunological reaction may be an antibody capture assay, an antigen capture assay or a sandwich assay. A detailed description of suitable immuno assays is given in "Antibodies, a laboratory manual" by E. Harlow and D. Lane, Cold Spring Harbor Laboratory, 1988, Chapter 14.

In a preferred test strip, antibodies may be found in two different zones of the porous carrier. From upstream to downstream: a first zone (31) with antibody which is provided with a label and which is freely movable in the porous carrier after contact with test liquid, and a second zone (32) with unlabelled antibody which is immobilised on the porous carrier. Antibodies are preferably monoclonal antibodies prepared according to routine methods or obtained commercially. In a preferred embodiment these monoclonal antibodies react at least with Candida albicans and/or Candida tropicalis and/or Candida parapsilosis and/or Candida glabrata.

The reagents can be introduced onto the test strip in a variety of ways. For example, it is possible to use printing processes known per se for this purpose. In this case, the reagents may either merely be applied to the surface of the test strip or impregnated into the test strip. It is sometimes also advantageous to introduce the reagents in a microencapsulated form. The binding reagents such as antibodies or avidin can be immobilised onto the test strip by covalent binding or absorption. These reagents can be immobilised as such or bound to particles, as for example latex particles. If the test strip consists, for example, of nitro-cellulose, antibodies can be coupled directly without a previous chemical treatment of the test strip. After coupling, however, the remaining binding sites on the test strip should be blocked with, for example, treatment with hydrophilic synthetic polymers, such as polyvinylalcohol, or hydrophylic biopolymers, such as human and bovine serum albumin, ovalbumin and the like. If the test strip consists of other materials such as paper, covalent coupling can be achieved with CNBr or carbonyldiimidazole.

A variety of labels has been used in immunoassays (see T.C.J. Gribnau et al. in the J. of Chromatography, 376, (1986) 175-189). Among them are indirect labels which require an additional reaction step before detection (e.g. enzymes) or direct labels (e.g. coloured particles) which do not require an additional step, but which can easily be detected by the naked eye, or if desired with the aid of a simple optical instrument. In a typical embodiment of the present invention visible particulate labels are used. In a preferred embodiment these particulate labels are gold or carbon sol particles. The preparation of these so particles as well as the preparation of antibody-so particle conjugates is among others described in EP 0 007 654, EP 0 032 270, WO 94/22011, US 5,529,901.

The labelled antibody is coated on the porous carrier in such a way that it is freely movable on the porous carrier when in the moist state, i.e. upon addition of test liquid. This is achieved by addition of blocking agents either to the conjugate buffer or to the porous carrier (except for the detection zone). Suitable blocking agents are for example proteins or detergents as used routinely in immunoassay buffers to prevent non-specific adsorption to the solid phase.

Unlabelled antibody is immobilised in the detection zone of the porous carrier and is not freely movable on the porous carrier when in the moist state. Immobilisation can either be achieved by covalent coupling or a simple absorption procedure. In the latter case care should be taken that no blocking agents, such as proteins or detergents, are present in the coating solution or on the detection zone of the porous carrier.

The porous carrier has preferably also a control zone (33) which generates a colour signal irrespective of whether the sample contains the analyte or not. Such a control zone contains for example immobilised antibodies, anti-antibodies or other binding reagents which react with the labelled antibodies.

According to the invention a separate container is provided in which the sample taker closely fits. The container may be dimensioned as a tube with an internal diameter only slightly larger than the largest diameter of the head of the means to collect and withdraw the sample and a depth sufficient to receive the sample taker in its entirety. The container should also be suited to hold an extraction fluid (50) in which the sample is to be dissolved or (re)suspended. This extraction fluid may be provided in the container but can also be added to the container either from an outside source or from a source contained within the device.

Various extraction fluids may be used for solubilisation or (re)suspension of the sample, such as phosphate or Tris buffers. Extraction fluid may also contain detergents, enzymes, acids or any other agent facilitating solubilisation or resuspension.

The device further contains means (60, 61, 62) to allow for the autonomous transport and application of material from the sample suspended in the extraction fluid (i.e. test liquid) onto the test strip. In a preferred embodiment, the test liquid migrates towards the housing of the device by gravitational force simply by placing the device on a horizontal plane. Protrusion (60) ensures that the separate container (40) assumes a position such that liquid is autonomously transported towards the housing. The housing may be equipped with openings (61) through which the test liquid may reach filter means (62) that may consist of sponge-like material. From there, the test liquid is transported further to the test strip (30) by capillary force. The test liquid is then transported through the strip by capillary action.

Any antigen which is present in the test liquid, reacts with the specific labelled antibodies from the first zone (31). The labelled antibody/antigen complex formed is then transported with the test liquid into the detection zone (32), where it is fixed by the immobilised antibodies. The antigen present in the test liquid can then be detected by reading the colour in the detection zone. Furthermore the test performance is controlled by observing the colour at the control spot (33), which always should give a positive result.

### Brief description of the figures

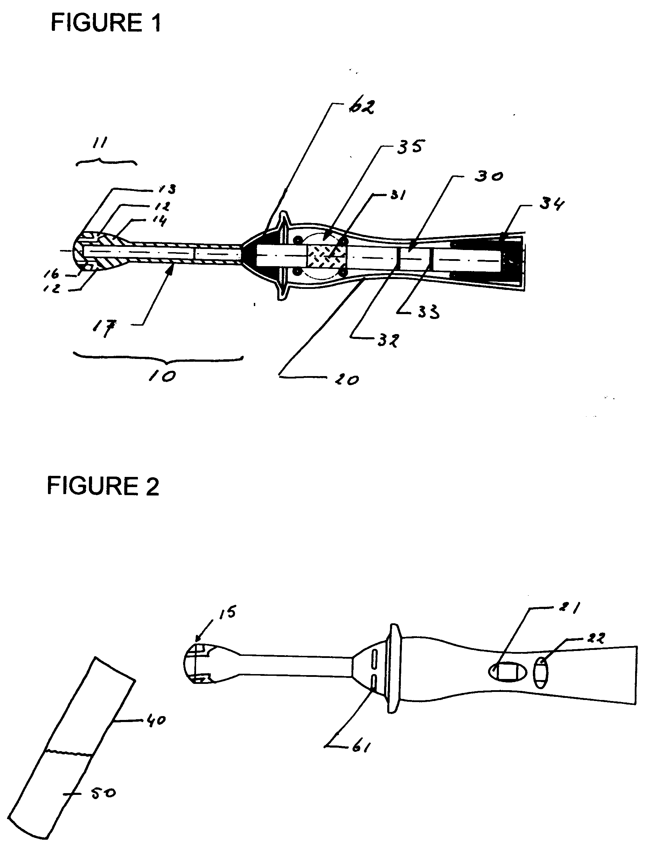

Figure 1 shows an open view of an embodiment of the analytical test device according to the invention. The device comprises a sample taker (10) with a longitudinal body (17) and means to collect and withdraw the sample (11) which comprise a head with openings (12) to obtain the sample and channels (16). Said sample taker is attached to a housing (20) comprising a test strip (30) of a porous carrier containing a first zone (31) with labelled antibody which is freely mobile when in the moist state, a second zone (32) with unlabelled antibody immobilised on the porous carrier and a control zone (33). The housing also contains a moisture-absorbing agent (35) and an absorber (34). Additionally, the housing contains a filter (62) to prevent any debris from contacting the test strip.

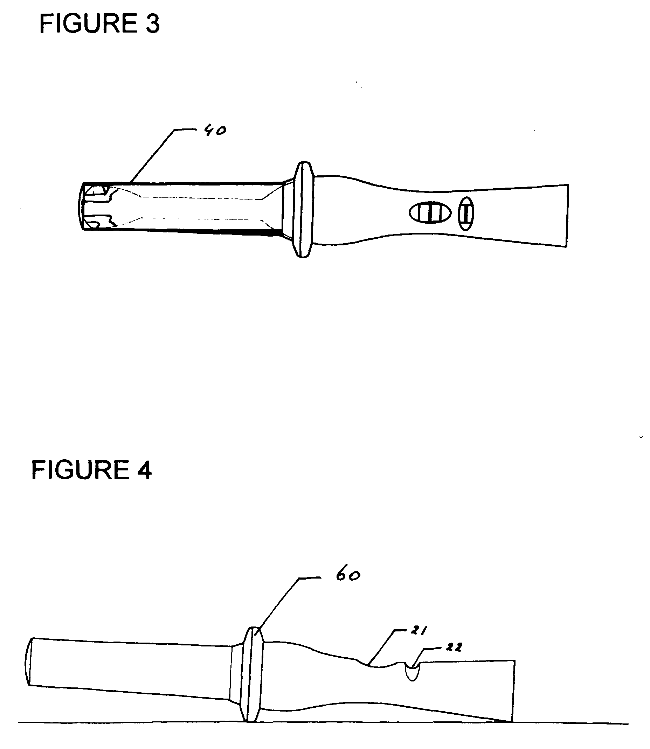

Figures 2 and 3 show a partially open view of the embodiment shown in figure 1 and an embodiment of the separate container (40) containing extraction fluid (50). The largest diameter (15) of the head of the means to collect and withdraw the sample is only slightly smaller than the inner diameter of the separate container. As a consequence, when the sample taker is inserted into the separate container and pushed all the way down as shown in figure 3, the extraction fluid will for the largest part be forced through the channels (16, figure 1) thereby suspending the sample in extraction fluid.
Figure 4 shows the embodiment shown in figure 1 when placed on a horizontal plane. The separate container automatically assumes a position in which the extraction fluid is autonomously transported towards the test strip. Through openings (61, figure 2) the fluid will reach the filter (62, figure 1) and from there be autonomously be transported towards the test strip. After completion of the immune reaction, the result can be read in opening 21, whereas the control spot, visible in opening 22 provides information on the reliability of the result.

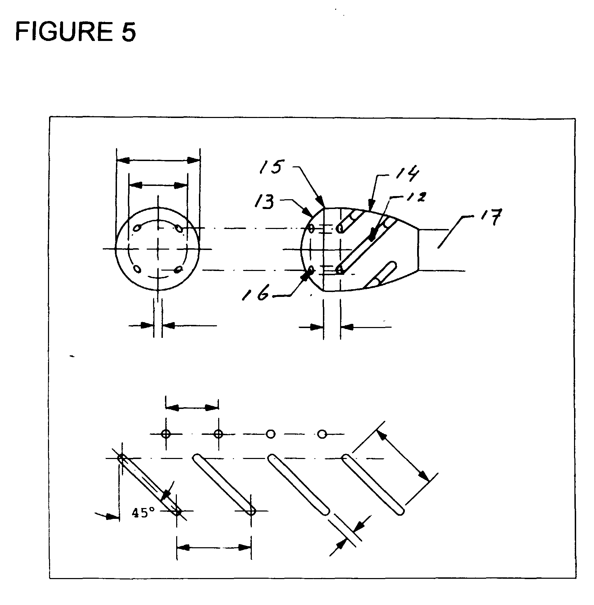

Figure 5 shows another embodiment of the means to collect and withdraw the sample (11 in figure 1), with the rounded head, thicker than the longitudinal body (17) of the sample taker with a front end (13) and a distal end (14) their borders being formed by the largest diameter of the head (15) with fluid channels (16) and notches (12).

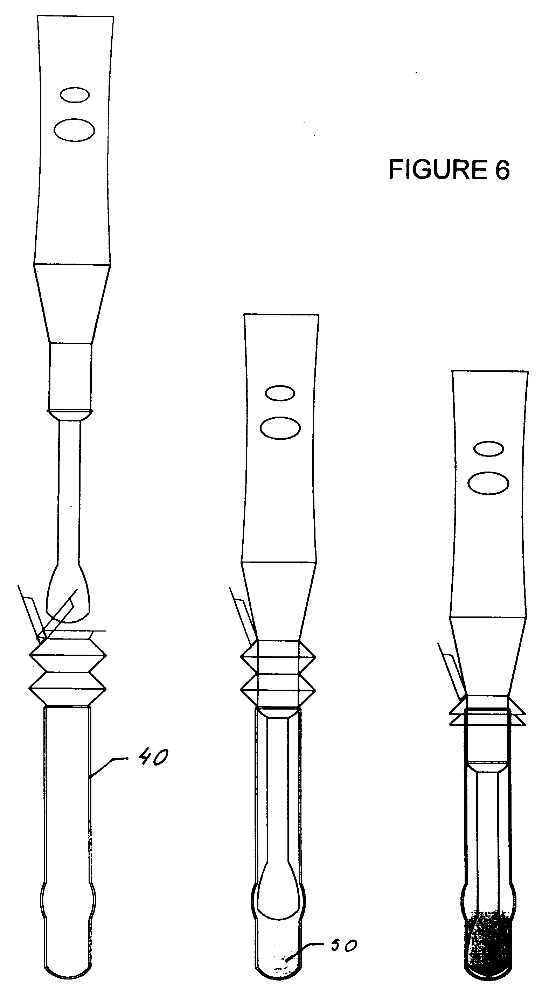

Figure 6 shows a partially open view of a further embodiment of an analytical test device according to the invention, the numbers corresponding to the elements mentioned in figures 1-5.

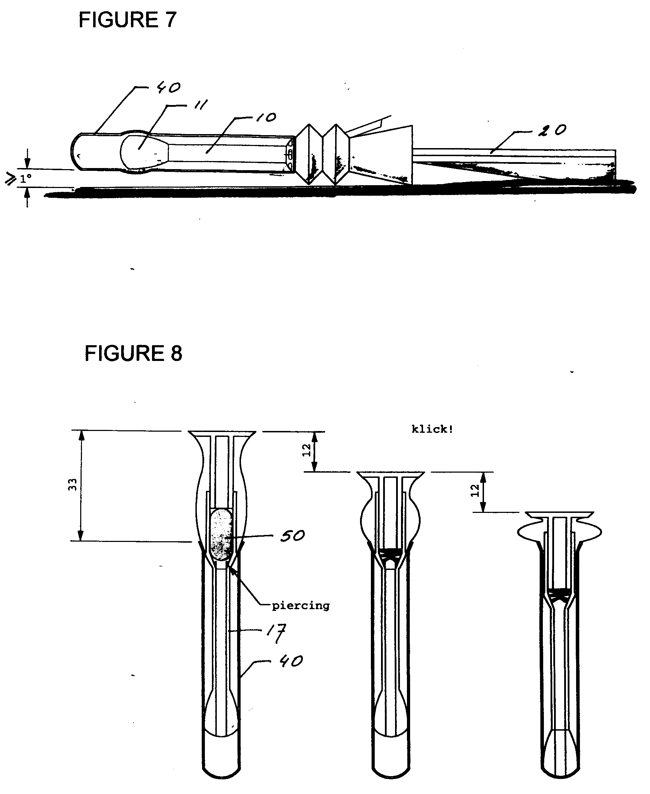

Figure 7 shows the embodiment shown in figure 6 when placed on a horizontal plane

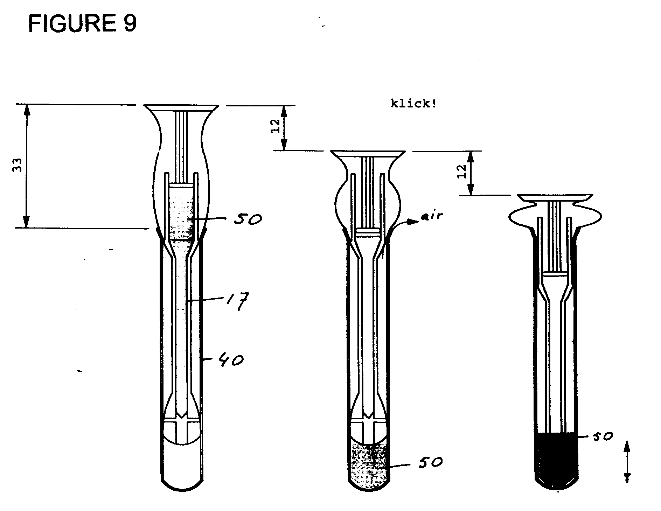

Figures 8 and 9 show yet another embodiment of an analytical test device according to the invention wherein the extraction fluid (50) is provided within the device. The extraction fluid is liberated from the device through the longitudinal body of the sample taker (17) into the separate container (40).

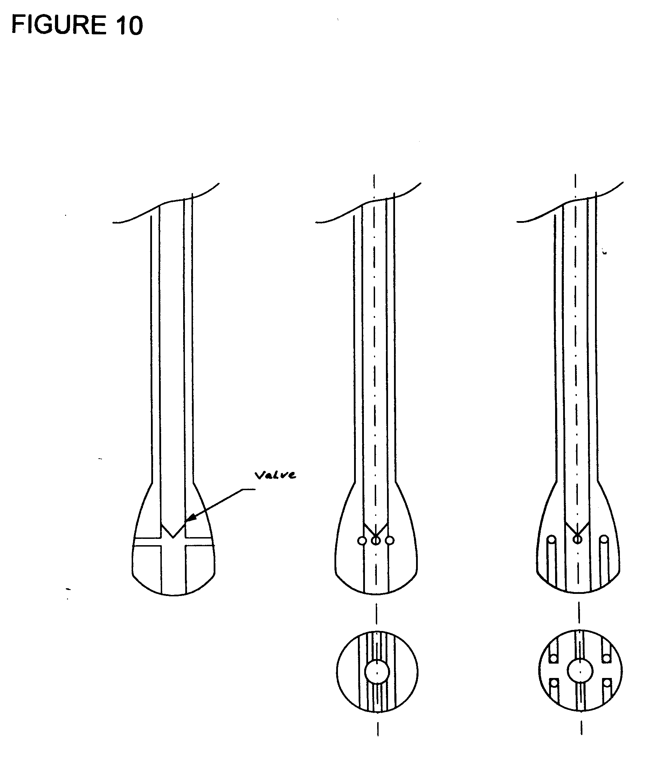

Figure 10 shows a longitudinal cross-section of the sample taker from the device shown in figure 9 containing a valve preventing back-flow of the extraction fluid.

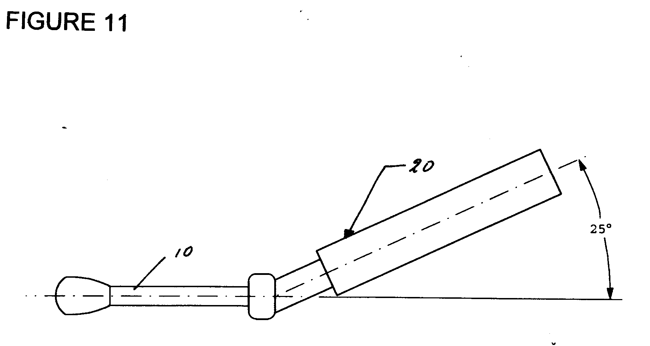

Figure 11 shows yet another embodiment of the device according to the invention where the housing (20) is attached to the sample taker (10) under a certain angle to facilitate manipulation of the device.

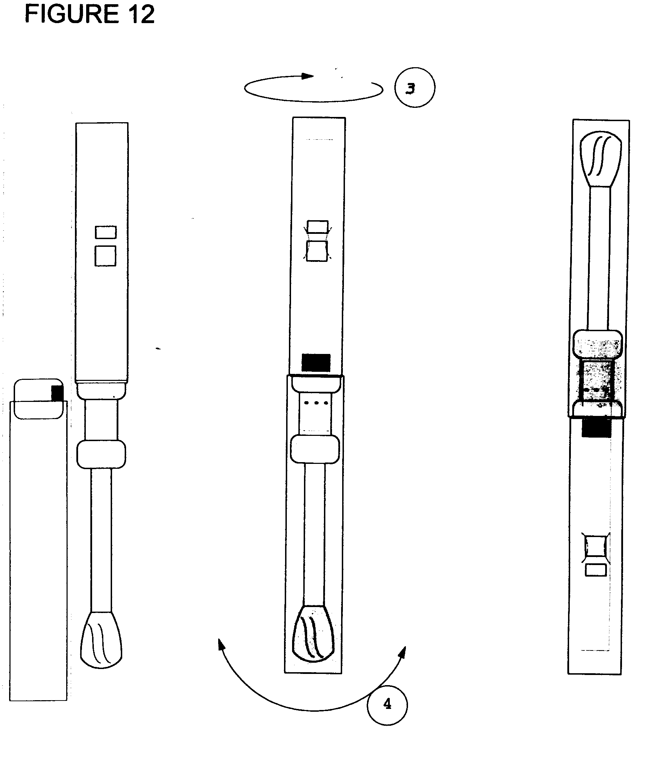

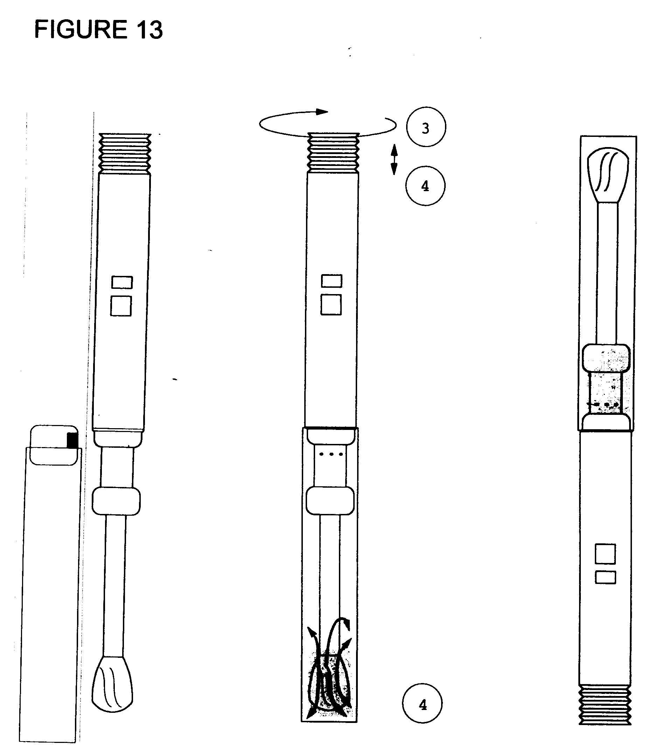

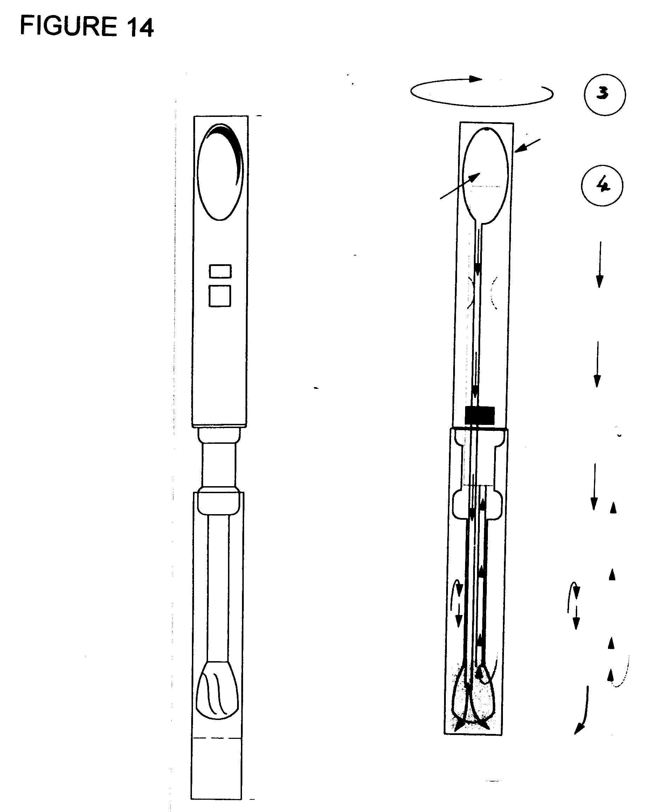

Figure 12-14 show three different other embodiments of the device according to the invention. In these devices the housing with the sample taker is screwed into the separate container (indicated by 3 in the figures). Sample is extracted by either shaking the device, as illustrated in figure 12 by arrow 4. Alternatively, extraction is performed by pumping the extraction fluid in and out the separate container, either by action of a plunger as indicated by the arrow 4 in figure 13 or by action of a balloon as indicated by 4 in figure 14.

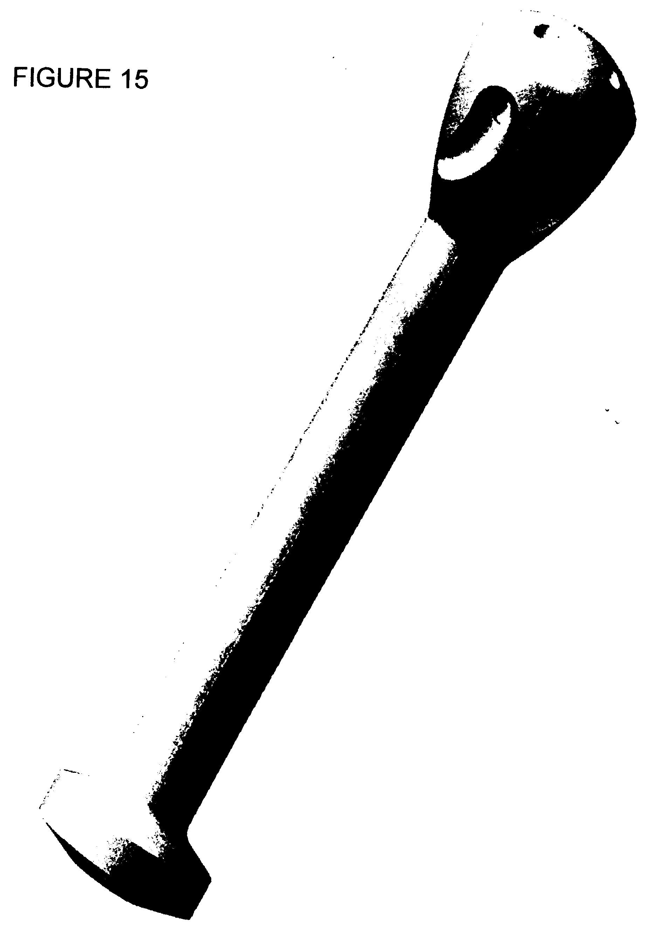

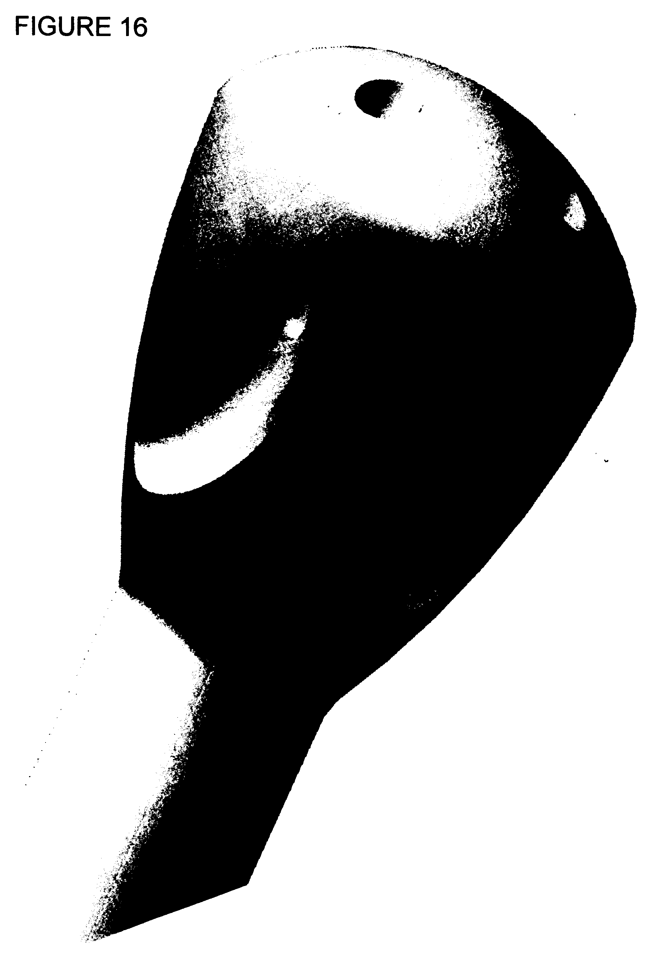

Figures 15 and 16 are three dimensional artist impressions of the means to collect and withdraw the sample (11) shown in figure 1.

The present invention will further be exemplified by the following examples, without limiting the invention.

### Examples

In a preferred embodiment, the device is used for the diagnosis of vulvovaginal Candidiasis. Vulvovaginal candidiasis (VVC) is a mucosal infection caused by Candida species. Candida albicans, a dimorphic commensal organism of the genital and gastrointestinal tracts, is the causative agent of VVC in approximately 85 to 90% of patients with positive vaginal fungal cultures. The remainder of the cases are due to other Candida species, the most common of which are Candida glabrata, Candida krusei, Candida parapsilosis and Candida tropicalis. An estimated 75% of all women will experience an episode of Candida vaginitis in their lifetime. In fact, vaginitis is among the most common clinical problems in women of childbearing age. Apart from Candida, Trichomonas vaginalis is the most common etiologic agent, whereas Hemophilus vaginalis and non-specific fungi are less frequent. Differentiation among these pathogens represents a significant aid in choosing proper treatment of cases of vaginitis

### Example 1. Monoclonal antibodies against Candida albicans

BALB/c mice were immunised intraperitoneally with 50 µg Candida albicans or Candidine (fragments of the Candida albicans cell-surface) diluted in Freunds Complete Adjuvans. After 1 month the mice were boosted intraperitoneally with 50 µg antigen diluted in Freunds Incomplete Adjuvans, while after 3 months the last immunisation was given intravenously. The immune response in the mice sera was checked with the Candiquant-Ig assay (Biomerica). Those mice which gave the highest response were used for fusion experiments.
Lymphocytes from mouse spleen cells were isolated and fused with myeloma cell line NS-1 at a ratio 1:1 using the standard PEG-fusion technique. Fused cells were resuspended in 96-well tissue culture plates containing Hypoxanthine-Aminoptereine-Thymidine selection medium. Hybrid clones producing Ig against Candida antigen were selected, cultured to 100% clonality, and stored in liquid nitrogen.

### Example 2. Specificity of monoclonal antibodies against Candida albicans

Both the monoclonal antibodies prepared under 1 (code nrs. OT-2B and OT-2F) and monoclonal antibodies obtained commercially from latron (code nrs. CA 4-2, CA 5-4, CA 1-2, CB 13-3), INSERM (code nr. 5B2), Instituto Superiore de Sanita (code nr. AF 1), and Montana State University (Mab B6.1) were tested on specificity in an ELISA. This ELISA test was performed as follows: a dilution series of various Candida species (Candida albicans A, B1 and B2, Candida parapsilosis and Candida tropicalis) was added to plates coated with rabbit-anti-Candida mannan IgG. (DAKO). After incubation and washing, one of the monoclonal antibodies described above was added. After incubation and washing, goat-anti-mouse-HRP (DAKO) was added as a label, followed by another incubation and washing step. Finally the enzyme activity was read after incubation with an enzyme substrate (TMB/H₂O₂) and stopping of the reaction with H₂SO₄.
Most monoclonal antibodies reacted with Candida albicans types A, B1 and B2, as well as with Candida tropicalis. Monoclonal antibody B6.1 seems to be the only antibody which recognises Candida glabrata as well.

### Example 3. Preparation of gold sol labelled monoclonal anti-Candida IgM solution (gold sol conjugate).

Gold sols with an average particle diameter of 50 nm (A540 = 5.0) were prepared according to the method described by Frens (Nature Physical Science Vo. 240, 1973, 20).
A solution of 1 mg monoclonal mouse anti-Candida IgM (code nr. AF 1) per ml sodium chloride (9 g/l) was adjusted to pH 9.0 using 0.1 M sodium hydroxide.
1 l of the gold sol solution was adjusted to pH 9.0 with 0.1 M sodium hydroxide, mixed with 20 ml of the monoclonal anti-Candida IgM solution and subsequently postcoated by adding 40 ml of a 20 M polyethylene glycol solution, pH 9.0. The postcoated gold sol conjugate was sedimented by centrifugation for 20 min. at 3500g at ambient temperature. After removing the supernatant by suction the gold sol pellet was resuspended to an A540 value of 50.0 in a solution containing 1% (v/v) calf serum, 160 g/l sucrose, 2% (w/v) Triton X100 and 3M Tris, pH 8.8.

### Example 4. Preparation of test strips.

On a rectangular sheet of glass paper measuring 100 mm in length and 7 mm in width, a first mobile reaction zone was formed by applying, along the width, 10 µl of a solution of gold sol conjugate (see under 3).
A second immobilised reaction zone (detection zone) was formed by pipetting , 40 mm from the bottom edge, 1 µl of a 1 mg/ml solution of monoclonal anti-Candida IgM solution (code no. AF 1) in 9 g/l sodium chloride, adjusted to pH 9.0 with 0.1 M NaOH.

A third reaction zone (control zone) was formed by pipetting, 50 mm from the bottom edge, 1 µl of a solution of 3.3 mg/ml monoclonal rat anti-mouse IgG solution (anti-kappa; code no. OT-1) in 9 g/l sodium chloride, adjusted to pH 9.0 with 0.1 M NaOH. The detection and control zones were subsequently air-dried (50° C).

### Example 5. Test performance.

### a) Sample collection

A sample taker as shown in figure 15 was constructed, pre-weighed and used to collect vaginal smears from symptomatic candidiosis patients. Sample takers were subsequently weighed in order to determine the weight of the sample. The vaginal smears were extracted by forcing the sample taker by means of a single stroke into a narrow tube which contained 1 ml of phosphate buffered saline. The thus obtained test fluid was then contacted with a test strip as described under example 4. The colour of the detection zone on the test strip was read after 4 min. Any colour remaining in the detection zone was read as positive and indicative for a vaginal Candida infection.

## Claims

1. Analytical test device specifically intended for home use for determining the presence or amount of an analyte in a sample taken from a body cavity, comprising:
a) a sample taker (10) with dimensions suited to fit into the body cavity and provided with means (11) to collect and withdraw the sample from the interior of the body cavity, said sample taker being attached to
b) a housing (20) for a test strip (30), said test strip comprising immunological means for detecting the analyte and
c) a separate container (40) in which the sample taker fits closely, suited to hold an extraction fluid (50) in such a way that the extraction fluid contacts the sample when the sample taker is fitted into the container and
d) means (60, 61, 62) to allow for the autonomous transport and application of material from the sample suspended in the extraction fluid onto the test strip.

2. The device according to claim 1 characterised in that said sample taker and said means to collect and withdraw the sample essentially consist of a non-absorbing, non-degradable, inert, synthetic material.

3. The device according to claim 2 characterised in that said synthetic material is polystyrene, polypropylene, polyethylene or ethylene vinylacetate.

4. The device according to claims 1-3 characterised in that the sample taker is dimensioned to be received in the vagina of a human female.

5. The device according to claims 1-4 characterised in that the means to collect and withdraw the sample (11) comprise a rounded head, thicker than the longitudinal body (17) of the sample taker, said head having a front end (13) and a distal end (14), said distal end being attached to the sample taker, the border between said ends being formed by the largest diameter of the head (15), wherein the distal end comprises notches (12) dimensioned to receive sufficient sample volume, said head also containing fluid channels (16) with openings at said front end of the head and at said notches, said openings at said notches being dimensioned such that the sample is released from the notches when fluid is applied under slight pressure at the opening of said channels at the front end of the head.

6. The device according to claims 1-5 characterised in that said immunological means are antibodies directed against Candida spec.

7. The device according to claims 1-6 characterised in that said test strip is a porous carrier through which a liquid is to be transported, said liquid comprising the analyte dissolved or suspended in extraction fluid, said carrier having an antibody specific for the analyte attached thereto, whereby part of said antibody is provided with a label and freely mobile within the porous carrier when in the moist state, and the other part of said antibody is immobilised in a detection zone on said porous carrier, the positioning of the labeled and immobilised antibody being such that test sample, when applied to the porous carrier, will first pick up the labelled antibody and thereafter will migrate to the detection zone. The labeled antibody and the freely mobile antibody may be of identical or of different specificity.

8. The device according to claim 7 characterised in that said label is a visible particulate label.

9. The device according to claim 8 characterised in that said label is a gold or carbon sol.

10. Use of the device according to any of the preceding claims in a diagnostic method.

11. Immunoassay for the detection or quantitation of analytes in samples taken from body cavities, comprising the steps of
a) providing a device according to any of the claims 1-9
b) collecting a sample from a body cavity with the sample taker
c) extracting that sample with buffer
d) contacting the extract with the test strip contained in the device
e) reading the result.
